Europäisches Patentamt

European Patent Office

Office européen des brevets

(11) Publication number: **0 332 753 B1**

## EUROPEAN PATENT SPECIFICATION

(45) Date of publication of patent specification: **05.08.92**  (51) Int. Cl.⁵: **C12M  1/28**

(21) Application number: **88120601.5**

(22) Date of filing: **09.12.88**

(54) **Improved device for performing microbiological culture tests.**

(30) Priority: **14.03.88 IT 1976888**

(43) Date of publication of application:
**20.09.89 Bulletin  89/38**

(45) Publication of the grant of the patent:
**05.08.92 Bulletin  92/32**

(84) Designated Contracting States:
**AT BE CH DE ES FR GB GR IT LI NL SE**

(56) References cited:
**FR-A- 2 381 102**
**FR-A- 2 612 297**
**US-A- 4 416 984**

(73) Proprietor: **S.p.A. ITALIANA LABORATORI BOUTY**
**Via Vanvitelli 6**
**I-20129 Milan(IT)**

(72) Inventor: **Ronchi, Gian Maria**
**C. so Milano 60**
**I-20030 Bovisio Masciago Milan(IT)**

(74) Representative: **Perani, Aurelio et al**
**c/o JACOBACCI-CASETTA & PERANI S.p.A 7,**
**Via Visconti di Modrone**
**I-20122 Milano(IT)**

EP 0 332 753 B1

## Description

The present invention relates to an improved device for performing microbiological culture tests, particularly bacteriological and/or mycological tests.

As is known, culture tests for determining the presence of bacteria use a nutrient substrate or a solid culture medium which, suitably inoculated, allows the formation of colonies of fungi or bacteria.

Said colonies must be sufficiently spaced from one another in order to facilitate their subsequent sampling to allow their identification and any tests for sensitivity to antibiotics or chemotherapeutic substances.

The inoculation of the solid culture medium occurs by immersing it in the liquid to be tested or in a solution thereof with subsequent incubation of the inoculated medium in sterile containers.

The danger of contamination of the culture medium is extremely important, as it would lead to an undesired growth thereon of micro-organisms not really present in the liquid to be tested.

In order to eliminate these disadvantages, various methods have been developed, including the "Castaneda method" which provides the presence, in the same sterile ampoule, of a liquid culture medium and of a solid one, the latter being fixed to a wall of the ampoule; this method, particularly usable for hemoculture, entails however some problems due first of all to the difficulty of removing from the ampoule samples of the colonies grown on the solid medium, as well as to the danger that by upsetting said ampoule the solid culture medium may be washed by the previously inoculated liquid medium, altering or removing the colonies of bacteria grown thereon.

A system using separate containers has been recently introduced, a first container containing a culture broth, which is inoculated with the liquid collected from the patient, and a second sterile container that can be attached tightly to the former one to allow the transfer of the inoculated culture broth to wet the solid culture medium contained in the latter one.

Though it has considerable advantages, this system requires the experience of qualified personnel, entails dangers or contamination during the step of attaching the container bearing the solid medium to the ampoule containing the culture broth, and requires accurate sterilization methods so that one often resorts to flame sterilization of the connecting collar provided on the broth-containing ampoule.

Furthermore, once the two containers are connected to one another it is not possible to separate them without risking the contamination of the media or the possible contamination of the operator.

Problems substantially similar to those described above for the Castaneda method arise in the case of upsetting the containers or of careless transport thereof.

The Italian patent No. 1 163 645, granted on April 8, 1987 and filed in the name of the same Applicant, discloses a device provided with a container body having a tank and an incubation chamber in which a solid culture medium is arranged, as well as valve means for hydraulically connecting the chamber and the tank or, alternatively, sealing them off from each other.

Though the device solved the above described problems, especially regarding the danger of contamination of the operator and of accidental washing of the solid medium by the liquid medium, in practical use it has been observed that it is susceptible to improvements.

For example, the actuation of the cover to open or close the valve was not always easy, and a deformation of the support of the solid medium was sometimes observed due to the sealing system.

The aim of the present invention is to provide an improved device for performing microbiological culture tests, particularly bacteriological and or mycological tests, which solves the above described problems especially with regard to the safety of the operator and its ease of operation.

Within the sphere of this task, an object of the invention is to provide a structurally simple and sturdy device that ensures absolute reliability as well as a modest manufacturing cost.

Another object of the invention is to further facilitate the use of the device so as to make it usable even by non-specialized personnel.

This task, as well as these and other goals which will become apparent hereinafter, are achieved by an improved device for performing microbiological culture tests, particularly of the bacteriological and mycological type, comprising a container defining a chamber and a tank, said chamber comprising a support for solid culture media, valve means being provided for a hydraulic connection between said chamber and said tank, said valve means being externally actuated to alternatively provide and cut off said connection, characterized in that said valve means comprises a piston element cooperating with a portion of said container to provide sealing

Further characteristics and advantages of the invention will become apparent from the description of a preferred but not exclusive embodiment of the improved device, illustrated by way of non-limitative example in the accompanying drawings, wherein:

figure 1 is a perspective view of the device according to the invention;

figure 2 is an exploded perspective view of the device;

figure 3 is a lateral elevation view, in longitudinal cross-section, with the valve means in closed position;

figure 4 is the same view as figure 3 but with the valve means in open position;

figures 5, 6 and 7 are sequential schematic views of the different steps of operation of the device.

With reference to the above described figures, the device according to the invention comprises a single-bodied container 1 having a first cylindrical portion 2 and a second cylindrical portion 3 connected to a truncated-cone portion 4, and the portions 2 and 3, respectively, define a tank and a chamber. To the open base of the tank 2 it is possible to apply a closure element 5 constituted by a double wall 6 for connection to the walls of tank 2, e.g. by means of a multi-start screw coupling and by a base 7 having an inner cylindrical element 8. The closure element 5 seals the tank 2, and furthermore has a hole 9 to which is applied applied a perforatable plug 10, known per se.

The inner cylindrical element 8 acts as housing for the stem 11 of a piston element 12 situated in the vicinity of the truncated-cone portion 4. The stem 11 furthermore has an external grip 13 and has slidable gaskets 14 which seal against the internal walls of the cylindrical element 8.

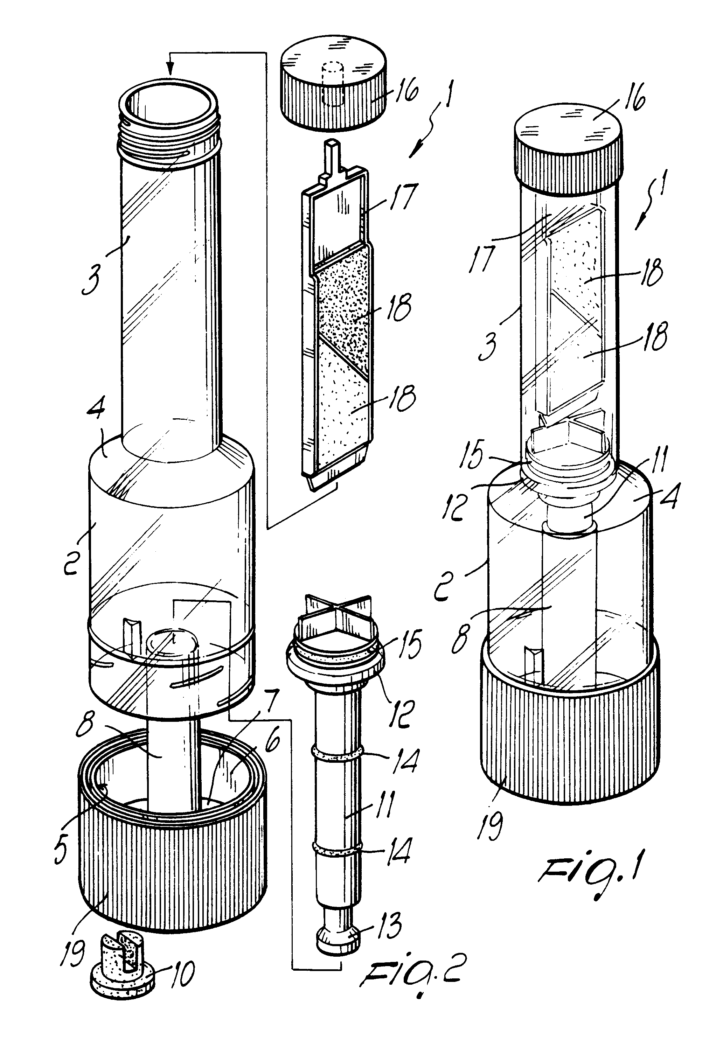

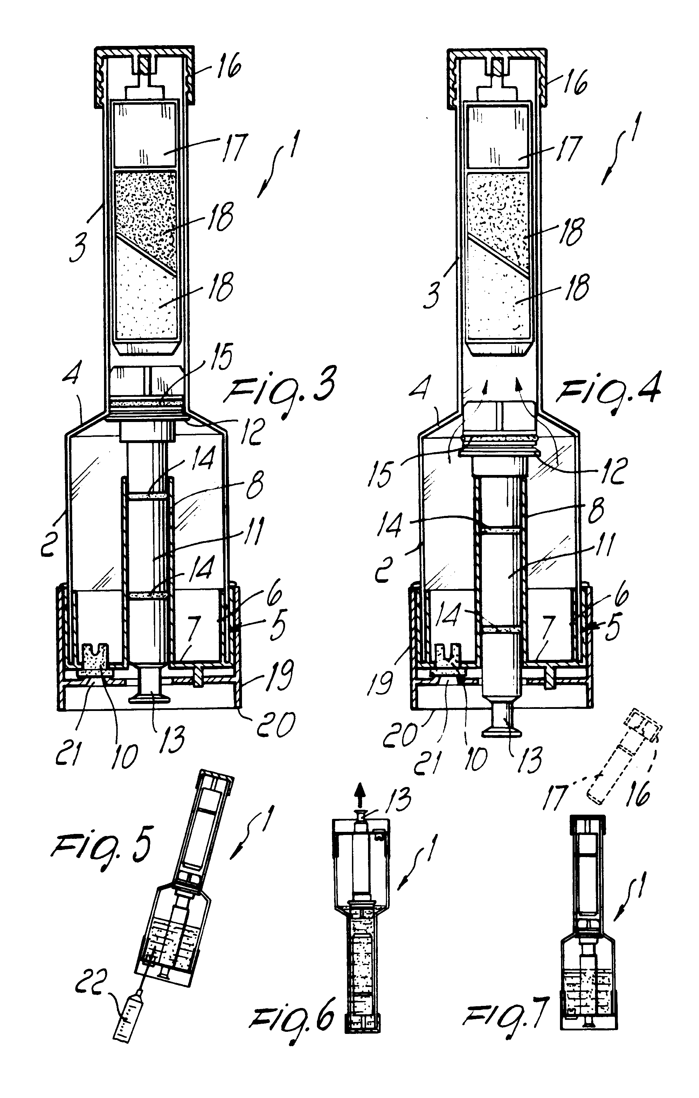

The piston element 12 has a gasket 15 suitable for sealing against the inner walls of the chamber 3 in particular, figure 3 illustrates the closure position of the piston element 12 which abuts against the top edge of the truncated-cone portion 4, while figure 4 illustrates the open position of the piston element 12 which is at a certain distance from the truncated-cone portion 4 so as to allow the passage of fluid from the tank 2 to the chamber 3.

The open base of chamber 3 is closed by an upper cap 16, preferably of the screw-on type, which bears a supporting plate 17 for one or more solid culture media 18. Naturally the upper plug 16 also performs a sealing closure of the chamber 3.

Advantageously, a lower cover 19 is applied over the closure element 5 and has a resting edge 20 so that the external grip 13 of the stem 11 does not protrude below when the piston element is in the closed position in this manner, it is possible to keep the container 1 in its normal vertical orientation when it rests on a plane surface. The lower cover 19 is furthermore preferably fitted tightly onto the closure element 5 and has a hole 21 at the perforatable plug 10, the function of the edges of the hole 21 being to retain the plug 10 in position. The operation of the device according to the invention is as follows.

A preset amount of dilution liquid and/or enrichment or selective enrichment broth is introduced into tank 2 during manufacture, the piston element 12 is in closed position and the chamber 3 contains the supporting plate 17 with one or more solid culture media 18.

The liquid sample to examine (serum, blood, urine or other liquid) is introduced by means of a syringe 22 through the perforatable plug 10 into tank 2, where it mixes with the liquid contained therein (figure 5).

The entire device is then incubated, if required, for the necessary time.

At the end of the incubation, the valve system constituted by the piston element 12 between chamber 3 and tank 2 is opened using the grip 13, and by turning the device upside down, the liquid is caused to pass into chamber 3, thus placing it in contact with solid culture media 18 contained therein (figure 6). At this point, the device is again turned upside down, causing the liquid to flow down into tank 2, and the valve is again closed (figure 7).

The device is subjected to further incubation to allow the growth of the colonies on the solid culture medium or media 18. At the end of this step and only in the case of growth of cultures, the cap 16 can be unscrewed to allow the removal of the support 17 with the solid culture media 18 and the possible sampling of bacterial colonies for further analyses (as illustrated in broken lines in same figure 7).

In practice it has been observed that the device according to the invention carries out the task and achieves the intended aim insofar as solid culture media have been inoculated with the liquid without having to open the device and, therefore, without any risk of contamination of the media or of the operator.

A great advantage of the invention is the extreme ease of actuating the valve means combined with an absolute reliability of the seal.

Another advantage is due to the transparency of the walls of the container, a feature which allows one to monitor the growth of the colonies on the media.

Not the last advantage is the possibility that the device can be used by non-specialized personnel due to its extreme simplicity and to the avoidance of the use of glass or of media to be prepared in the laboratory whether it be for dilutions or for inoculations.

**Claims**

1.  Improved device for performing microbiological culture tests, particularly of the bacteriological and mycological type, comprising a container defining a chamber and a tank, said chamber comprising a support for solid culture media, valve means being provided for a hydraulic connection between said chamber and said tank, said valve means being externally actuated to alternatively provide and cut off said connection, characterized in that said valve means comprises a piston element cooperating with a portion of said container to provide sealing.

2. Device according to claim 1, characterized in that said container has a first cylindrical portion connected to a second cylindrical portion having a smaller diameter by means of a truncated-cone portion, said container being single-bodied.

3. Device according to claim 1 or 2, characterized in that said first cylindrical portion defines said tank and is closed below by a closure element having an inner cylindrical element suitable for supporting said piston element, said closure element furthermore having a through hole sealed off by a plug which can be perforated to inject a liquid to be tested into said tank.

4. Device according to claim 3, characterized in that it comprises a lower cover applicable to said closure element and having an edge for the resting of said container and a hole for access to said perforatable plug said hole designed to retain said perforatable plug in position.

5. Device according to one or more of the preceding claims, characterized in that said piston element has a stem slideable in said inner cylindrical element and comprising at least one sealing gasket, said stem furthermore having a grip external to said closure element for the actuation of said piston element.

6. Device according to one or more of the preceding claims, characterized in that said piston element has two positions, a closure position in which said piston abuts against said truncated-cone portion, an open position in which said piston element is spaced from said truncated-cone portion so that said tank and said chamber are hydraulically connected, said second cylindrical portion with smaller diameter defining said chamber.

7. Device according to one or more of the preceding claims, characterized in that said piston element comprises a gasket suitable for providing a seal against the inner wall of said second cylindrical portion near the top of said truncated-cone portion.

8. Device according to one or more of the preceding claims, characterized in that said tank is provided by said first cylindrical portion and in that said chamber is provided by said second cylindrical portion.

9. Device according to one or more of the preceding claims, characterized in that said chamber is closed on the side opposite to said piston element by an upper cap, said upper cap bearing a plate underneath it for supporting said solid media.

10. Device according to one or more of the preceding claims, characterized in that said upper cap is screwed to said second cylindrical portion and is removable together with said plate supporting said solid media.

11. Device according to one or more of the preceding claims, characterized in that said tank contains a dilution liquid.

12. Device according to one or more of the preceding claims, characterized in that said tank contains a liquid constituting an enrichment medium.

13. Device according to one or more of the preceding claims, characterized in that said tank contains a dilution and/or enrichment or selective enrichment liquid.

14. Device according to one or more of the preceding claims, characterized in that said plate supports a plurality of solid culture media.

**Revendications**

1. Dispositif perfectionné pour effectuer des tests de cultures microbiologiques, notamment du type bactériologique et du type mycologique, comportant un récipient qui forme une chambre et un réservoir, ladite chambre comportant un support pour des milieux de cultures solides, des moyens à soupape étant prévus pour assurer une liaison hydraulique entre ladite chambre et ledit réservoir, lesdits moyens à soupape étant actionné de l'extérieur pour établir et couper alternativement ladite liaison, caractérisé en ce que lesdits moyens à soupape comportent un élément à piston coopérant avec une partie dudit récipient pour assurer l'étanchéité.

2. Dispositif selon la revendication 1, caractérisé en ce que ledit récipient comporte une première partie cylindrique reliée par une partie tronconique à une deuxième partie cylindrique ayant un plus faible diamètre, ledit récipient ayant un seul corps.

3. Dispositif selon la revendication 1 ou 2, caractérisé en ce que ladite première partie cylindrique forme ledit réservoir et est fermée en bas par un élément de fermeture comportant un élément cylindrique intérieur approprié au support dudit élément à piston, ledit élément de fermeture présentant en outre un trou de traversée fermé de façon étanche par un bouchon qui peut être percé pour injecter un liquide à examiner dans ledit réservoir.

4. Dispositif selon la revendication 3, caractérisé en ce qu'il comporte un couvercle inférieur pouvant être disposé sur ledit élément de fermeture et présentant un bord pour l'appui dudit récipient ainsi qu'un trou d'accès audit bouchon pouvant être percé, ledit trou étant agencé pour maintenir en position ledit bouchon pouvant être percé.

5. Dispositif selon l'une ou plusieurs des revendications précédentes, caractérisé en ce que ledit élément à piston comporte une tige pouvant coulisser dans ledit élément cylindrique intérieur et munie d'au moins un joint d'étanchéité, ladite tige comportant en outre une poignée située à l'extérieur dudit élément de fermeture et destinée à la manoeuvre dudit élément à piston.

6. Dispositif selon l'une ou plusieurs des revendications précédentes, caractérisé en ce que ledit élément à piston peut prendre deux positions : une position de fermeture dans laquelle ledit piston bute contre ladite partie tronconique et une position d'ouverture dans laquelle ledit élément à piston est espacé de ladite partie tronconique de manière que ledit réservoir et ladite chambre soient en liaison hydraulique, ladite deuxième partie cylindrique de plus faible diamètre formant ladite chambre.

7. Dispositif selon l'une ou plusieurs des revendications précédentes, caractérisé en ce que ledit élément à piston comporte un joint convenant pour assurer l'étanchéité contre la paroi intérieure de ladite deuxième partie cylindrique à proximité du haut de ladite partie tronconique.

8. Dispositif selon l'une ou plusieurs des revendications précédentes, caractérisé en ce que ledit réservoir est formé par ladite première partie cylindrique et en ce que ladite chambre est formée par ladite deuxième partie cylindrique.

9. Dispositif selon l'une ou plusieurs des revendications précédentes, caractérisé en ce que ladite chambre est fermée par un capuchon supérieur du côté situé à l'opposé dudit élément à piston, ledit capuchon supérieur portant, sous lui, une plaque de support desdits milieux solides.

10. Dispositif selon l'une ou plusieurs des revendications précédentes, caractérisé en ce que ledit capuchon supérieur est vissé sur ladite deuxième partie cylindrique et peut être retiré conjointement avec ladite plaque de support desdits milieux solides.

11. Dispositif selon l'une ou plusieurs des revendications précédentes, caractérisé en ce que ledit réservoir contient un liquide de dilution.

12. Dispositif selon l'une ou plusieurs des revendications précédentes, caractérisé en ce que ledit réservoir contient un liquide consitutant un milieu d'enrichissement.

13. Dispositif selon l'une ou plusieurs des revendications précédentes, caractérisé en ce que ledit réservoir contient un liquide de dilution et/ou d'enrichissement ou d'enrichissement sélectif.

14. Dispositif selon l'une ou plusieurs des revendications précédentes, caractérisé en ce que ladite plaque porte une pluralité de milieux de cultures solides.

**Patentansprüche**

1. Verbesserte Vorrichtung zum Durchführen mikrobiologischer Tests an Kulturen, insbesondere der bakteriologischen und mykologischen Art, mit einem Behälter, der eine Kammer und einen Tank eingrenzt, wobei die Kammer einen Träger für ein festes Kulturmedium aufweist, Ventilmittel zum Herstellen einer hydraulischen Verbindung zwischen der Kammer und dem Tank vorgesehen sind und die Ventilmittel extern zum alternativen Herstellen und Absperren der genannten Verbindung betätigt werden, dadurch **gekennzeichnet,** daß die Ventilmittel ein Kolbenelement umfassen, welches mit einem Abschnitt des Behälters zusammenwirkt, um eine Dichtung vorzusehen.

2. Vorrichtung nach Anspruch 1, dadurch **gekennzeichnet,** daß der Behälter einen ersten zylindrischen Abschnitt aufweist, der mit einem zweiten zylindrischen Abschnitt kleineren Durchmessers mittels eines kegelstumpfförmigen Abschnittes verbunden ist, wobei der Behälter einteilig ist.

3. Vorrichtung nach Anspruch 1 oder 2, dadurch **gekennzeichnet,** daß der erste zylindrische Abschnitt den genannten Tank bildet und unten durch ein Abschlußelement mit einem inneren zylindrischen Element zum Unterstützen des Kolbenelementes geschlossen ist, wobei das Abschlußelement ferner eine mittels eines Stopfens verschlossene Durchgangsbohrung aufweist, die zum Einspritzen einer zu testenden Flüssigkeit in den Tank perforiert werden kann.

4. Vorrichtung nach Anspruch 3, dadurch **gekennzeichnet,** daß sie einen unteren Deckel umfaßt, der auf das Abschlußelement aufsetzbar ist und einen Rand für das Aufsetzen des Behälters sowie ein Loch als Zugang zu dem perforierbaren Stopfen hat, wobei das Loch so gestaltet ist, daß es den perforierbaren Stopfen in Position hält.

5. Vorrichtung nach einem oder mehreren der vorangehenden Ansprüche, dadurch **gekennzeichnet,** daß das Kolbenelement einen in dem inneren zylindrischen Element gleitbaren Schaft mit mindestens einer Dichtung umfaßt, wobei der Schaft ferner einen außerhalb des Abschlußelementes gelegenen Griff zur Betätigung des Kolbenelementes aufweist.

6. Vorrichtung nach einem oder mehreren der vorangehenden Ansprüche, dadurch **gekennzeichnet,** daß das Kolbenelement zwei Stellungen aufweist, nämlich eine Schließstellung, in welcher der Kolben an dem kegelstumpfförmigen Abschnitt anliegt, und eine Offenstellung, in welcher das Kolbenelement von dem kegelstupfförmigen Abschnitt einen Abstand hat, so daß der Tank und die Kammer hydraulisch verbunden sind, wobei der zweite zylindrische Abschnitt kleineren Durchmessers die genannte Kammer bildet.

7. Vorrichtung nach einem oder mehreren der vorangehenden Ansprüche, dadurch **gekennzeichnet,** daß das Kolbenelement eine Dichtung zum Schaffen einer Abdichtung zwischen der Innenwand des zweiten zylindrischen Abschnittes nahe dem oberen Ende des kegelstumpfförmigen Abschnittes aufweist.

8. Vorrichtung nach einem oder mehreren der vorangehenden Ansprüche, dadurch **gekennzeichnet,** daß der Tank vom ersten zylindrischen Abschnitt gebildet ist und daß die Kammer vom zweiten zylindrischen Abschnitt gebildet ist.

9. Vorrichtung nach einem oder mehreren der vorangehenden Ansprüche, dadurch **gekennzeichnet,** daß die Kammer auf der dem Kolbenelement gegenüberliegenden Seite durch eine obere Kappe verschlossen ist, unter welcher eine Platte zum Abstützen des festen Mediums angeordnet ist.

10. Vorrichtung nach einem oder mehreren der vorangehenden Ansprüche, dadurch **gekennzeichnet,** daß die obere Kappe mit dem zweiten zylindrischen Abschnitt verschraubt und zusammen mit der das feste Medium unterstützenden Platte entfernbar ist.

11. Vorrichtung nach einem oder mehreren der vorangehenden Ansprüche, dadurch **gekennzeichnet,** daß der Tank eine Verdünnungsflüssigkeit enthält.

12. Vorrichtung nach einem oder mehreren der vorangehenden Ansprüche, dadurch **gekennzeichnet,** daß der Tank eine Flüssigkeit enthält, welche ein Anreicherungsmedium bildet.

13. Vorrichtung nach einem oder mehreren der vorangehenden Ansprüche, dadurch **gekennzeichnet,** daß der Tank eine Verdünnungs-/oder Anreicherungsflüssigkeit oder wahlweise eine Anreicherungsflüssigkeit enthält.

14. Vorrichtung nach einem oder mehreren der vorangehenden Ansprüche, dadurch **gekennzeichnet,** daß die Platte eine Vielzahl von festen Kulturmedien unterstützt.

Fig.1

Fig.2

Fig.3

Fig.4

Fig.5

Fig.6

Fig.7